# EUROPÄISCHE PATENTSCHRIFT

(11) **EP 1 272 151 B1**
(45) Veröffentlichungstag und Bekanntmachung des Hinweises auf die Patenterteilung: **01.08.2007**
(21) Anmeldenummer: 01923709.8
(22) Anmeldetag: 31.03.2001
(51) Int. Cl.: A61K 8/86, A61K 8/25, A61Q 11/00

(54) **BELAGHEMMENDES FLÜSSIGES ZAHNREINIGUNGSMITTEL**
PLAQUE-CONTROLLING LIQUID TOOTH CLEANING AGENT
DENTIFRICE LIQUIDE ANTI-PLAQUE

(30) Priorität: 11.04.2000 DE 10017997
(43) Veröffentlichungstag der Anmeldung: 08.01.2003
(73) Patentinhaber: Henkel Kommanditgesellschaft auf Aktien, 40589 Düsseldorf (DE)
(72) Erfinder: LEINEN, Hans, Theo, 40229 Düsseldorf (DE); GREGORI, Dorothea, 41470 Neuss (DE)
(86) Internationale Anmeldenummer: PCT/EP2001/003698
(87) Internationale Veröffentlichungsnummer: WO 2001/076548

(56) Entgegenhaltungen:
- EP-A- 0 549 287
- WO-A-94/23691
- DE-A- 19 845 247
- FR-A- 2 684 550
- GB-A- 2 227 661

## Beschreibung

Die Erfindung betrifft flüssige Zahnreinigungsmittel mit einem Gehalt an Poliermitteln, Feuchthaltemitteln, belaghemmenden antimikrobiellen Stoffen und Wasser, die weitgehend transparent bis klar sind und ein günstiges rheologisches Verhalten für die Dosierung aus flexiblen Kunststofffläschchen aufweisen.

Es sind bereits flüssige Zahnreinigungsmittel bekannt, die sich aus flexiblen Kunststofffläschchen unter leichtem Druck ausbringen lassen. Solche Produkte, wie sie z. B. in WO 94/01080 A2 beschrieben sind, weisen ein weißes bis stark trübes Aussehen auf. Aus DE 2033678 B2 waren Zahnpasten bekannt, die ein Kieselsäure-Poliermittel und eine Feuchthaltemittel-Kombination mit einem Gehalt an Polyethylenglycolen mit Molekulargewichten im Bereich von 800 bis 2000 enthalten. Diese Produkte weisen ein klar durchsichtiges bis transparentes Aussehen auf. In EP 0754027 B2 sind transparente flüssige Zahnpflegemittel beschrieben, die eine Kieselsäure-Poliermittel und eine Feuchthaltemittel-Kombination aus Sorbit und einem niedermolekularen Polyethylenglycol enthalten.

Das Dokument DE-19845247 offenbart flüssige Zahnreinigungsgele mit einer Viskosität von 10-100 Pa.s. enthaltend 10-15 Gew.% Kieselsäure-Poliermitteln (Sident), 55-70 Gew.% Feuchthaltemittel im bestimmte Gewichtsverhältnis von "10 : 7/8 : 0,5/1,5" Sorbit, Glycerin und Polyethylenglykol, 15-30 Gew.% Wasser, weniger als 10 Gew.% weitere Zahnpasteninhaltstoffe, und den optionalen Einsatz von antimikrobiellen Stoffen z.B. Triclosan und Hexetidin, und möglicherweise Polyethylenglykole mit Molekulargewicht von 1500- 1.000.000 als Bindemittel.

Die Aufgabe der vorliegenden Erfindung bestand darin, wäßrige, flüssige Zahnreinigungsgele mit guter Transparenz und mit einem Gehalt an antimikrobiellen Wirkstoffen zur Bekämpfung von Zahnbelag zur Verfügung zu stellen. Es wurde gefunden, daß sich sowohl die flüssige Konsistenz als auch eine gute Transparenz erreichen lassen, wenn die Produkte weniger als 50 Gew.-% Sorbit und 0,1 - 5 Gew.-% eines Polyethylenglycols mit einem mittleren Molgewicht von 800 bis 10.000 und 0,1 - 1 Gew.-% eines antimikrobiellen Wirkstoffs, ausgewählt aus Triclosan, Hexetidin und Gemischen davon enthalten.

Gegenstand der Erfindung ist daher ein wäßriges flüssiges Zahnreinigungsgel mit einer Viskosität unterhalb 50 Pa.s (20°C), gekennzeichnet durch einen Gehalt von 10-20 Gew.% eines Kieselsäure-Poliermittels

| | |
|---|---|
| 40-60 Gew.-% | einer Feuchthaltemittel-Kombination, bestehend aus weniger als 50 Gew.-% Sorbit (bezogen auf das gesamte Mittel) weniger als 10 Gew.-% Glycerin und 1-5 Gew.-% Polyethylenglycol mit einem mittleren Molekulargewicht von 800-10000 |
| 0,1-1 Gew.-% | eines antimikrobiellen Wirkstoffs, ausgewählt aus Triclosan, Hexetidin und Gemischen davon, |
| 1-10 Gew.% | weiterer Zahnpasteninhaltsstoffe und |
| 25-35 Gew.-% | Wasser |

Als flüssig, im Sinne der Erfindung, werden dabei solche Zubereitungen verstanden, deren Viskosität unter 50 Pa·s (bei 20°C und bei einer Schergeschwindigkeit von D = 4S⁻¹ bei Messung in einem Rotationskosimeter des Typs Brookfield RVF mit Spindel 3 bzw. 4 bei 4rpm) liegt und die sich aus einem flexiblen Spenderfläschchen durch leichten Druck dosieren lassen. Durch die erfindungsgemäße Viskosität wird ein langsames Einsinken des Gels in die Borsten der Zahnbürste bewirkt.

Die Transparenz des Zahnreinigungsgels sollte wenigstens so hoch sein, daß durch eine etwa 1 cm dicke Schicht des Zahngels (gefüllt in eine Küvette von 1x1x4 cm Kantenlänge) eine Schrift mit ca. 4 mm Buchstabenhöhe und ca. 3 mm Buchstabenbreite noch gut lesbar ist. Die erfindungsgemäße Viskosität und Transparenz werden durch Auswahl und Menge der verwendeten Polierkomponenten, Verdickungsmittel und Feuchthaltemittel erzielt.

Als Kieselsäure-Polierkomponenten eignen sich alle als Putzkörper bekannten Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten, wie sie z.B. aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sog. Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10-20 Gew.-% des Zahngels. Bevorzugt geeignete Fällungskieselsäuren dieser Art weisen außerdem gerundete Ecken und Kanten auf und sind unter der Handelsbezeichnung Sident^{®} 12 DS (DEGUSSA) erhältlich. Eine weitere geeignete Kieselsäure ist Zeodent 113 (Huber Corp) mit einer BET-Oberfläche von 150-250 m²/g.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 -14 µm bei einer spezifischen Oberfläche von 40 - 75 m²/g (nach BET) aus und eignen sich besonders gut für flüssige Zahngele gemäß der vorliegenden Erfindung.

Weitere Poliermittel, insbesondere solche, die einen Brechungsindex aufweisen, der von 1,45 um mehr als 0,1 abweicht, sind bevorzugt nicht oder allenfalls in Mengen von weniger als 2 Gew.-% enthalten. Solche Poliennittelkomponenten sind z.B. Bims, Zirkoniumsilikat oder Gamma-Aluminiumoxid.

Von entscheidender Bedeutung für die Rheologie und Transparenz der erfindungsgemäßen flüssigen Zahnreinigungsgele ist die Zusammensetzung der flüssigen Trägerphase aus Wasser und Feuchthaltemitteln. Die Gesamtmenge von 40-60 Gew.-% der Feuchthaltemittel-Kombination bezieht sich auf das gesamte Zahnreinigungsgel und besteht im wesentlichen aus Sorbit und Polyethylenglycol. Die Menge an Sorbit stollte aber unterhalb von 50 Gew.-% liegen, gegebenenfalls kann Glycerin in Mengen von weniger als 10 Gew.-% enthalten sein. Als Polyethylenglycol eignen sich die höhermolekularen Typen mit mittleren Molgewichten von 800 bis 10 000, bevorzugt von 1000 bis 4000. Der Wassergehalt der erfindungsgemäßen Zahngele beträgt 25-35 Gew.-% und ergibt sich als Summe der durch die Rohstoffe wie z.B. 70 %-iges Sorbit oder 86%-iges Glycerin eingetragenen und der separat zugesetzten Wassermengen. Die angegebenen Mengen an Sorbit und Glycerin beziehen sich auf eine wasserfreie Aktivsubstanz.

Als antimikrobielle Wirksoffe zur Hemmung der Bildung von Plaque eignen sich besonders 2.2,4' - Trichlor-2' -hydroxy-diphenylehter (Triclosan) oder 1,3-Bis-(2-ethylhexyl)-5-methyl-5-amino-hexahydropyrimidin (Hexetidin) oder Gemische dieser antimikrobiellen Komponenten. Das Hexetidin kann auch in Form eines wasserlöslichen Salzes, z.B. des Benzoats, des Terephthalats oder des Salicylats enthalten sein. Diese ausgewählten antimikrobiellen Wirkstoffe lassen sich besonders leicht klar und stabil in die erfindungsgemäßen Zahngele einarbeiten.

Zusätzlich zu den genannten obligatorischen Komponenten können die erfindungsgemäßen Zahnreinigungsgele 1-10 Gew.-% weiterer Zahnpasteninhaltsstoffe enthalten, die in Art und Menge die Transparenz nicht negativ beeinflussen. Solche Inhaltsstoffe sind z.B. Bindemittel, Tenside, Geschmacks- und Süßstoffe, Antizahnwirkstoffe, Fluorverbindungen, Vitamine, Panthenol und andere Wirksubstanzen.

Als Bindemittel können z.B. natürliche und/oder synthetische wasserlösliche Polymere wie Alginate, Carragheenate, Traganth, Stärke und Stärkeether, Celluloseether wie z.B. Carboxymethylcellulose (Na-Salz), Hydroxyethylcellulose, Methylhydroxypropylcellulose, Guar, Akaziengum, Agar-Agar, Xanthan-Gum, Succinoglycan-Gum. Johannisbrotmehl, Pectine, wasserlösliche Carboxyvinylpolyrnere (z.B. Carbopol^{®}-Typen), Polyvinylalkohol. Polyvinylpyrrolidon, Polyethylenglycole, insbesondere solche mit Molekulargewichten von 1500 - 1 000 000 eingesetzt werden.

Besonders gut geeignete Bindemittel sind Xanthan-Gum, Carboxymethylcellulose, Polyvinylpyrrolidon und Gemische dieser wasserlöslichen Polymeren, die in einer Menge bis zu 1.0 Gew.-% enthalten sein können.

Geeignete Tenside sind z.B. lineare Natriumalkylsulfate mit 12 - 18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12 - 16 C-Atomen in der linearen Alkylgruppe und 2 - 6 Glycolethergruppen im Molekül, von linearem Alkan(C₁₂-C₁₈)-Sulfonat, von Sulfobernsteinsäuremonoalkyl(C₁₂-C₁₈)-estern, von sulfatierten Fettsäuremonoglyceriden, sulfatierten Fettsäurealkanolamiden, Sulfoessigsäurealkyl(C₁₂-C₁₆)-estern, Acylsarcosinen, Acyltauriden und Acylisethionaten mit jeweils 8 - 18 C-Atomen in der Acylgruppe.

Weiterhin eignen sich auch ampholytische und zwitterionische Tenside, insbesondere Betain-Tenside wie z.B. Kokosalkylbetain oder Kokosacylamidopropyl-Betain. Schließlich können auch nichtionische Tenside eingesetzt werden, z.B. Oxethylate von Fettsäuremono-und diglyceriden, von Fettsäure-Sorbitanestem und Alkyl(oligo)-glucoside.

In einer bevorzugten Ausführung enthalten die erfindungsgemäßen Zahnreinigungsgele als weitere Inhaltsstoffe ein wasserlösliches Bindemittel und eine Tensidkombination aus anionischen, zwitterionischen und nichtionischen Tensiden. Bevorzugt sind 0,5-3 Gew.-% der Tenside enthalten, das Mengenverhältnis von anionischen zu zwitterionischen und nichtionischen Tensiden ist bevorzugt 1: (0,2-1).

Als Geschmackstoffe werden üblicherweise Aromen und Süßungsmittel verwendet. Geeignete Aromen sind z.B. Pfefferminzöl. Krauseminzöl, Eukalyptusöl, Anisol, Fenchelöl. Kümmelöl, Methylacetat, Zimtaldehyd, Anethol, Vanillin, Thymol und andere natürliche oder naturidentische ätherische Öle oder auch synthetische Geschmackstoffe.

Als Süßungsmittel eignen sich z.B. Saccharin-Natrium. Acesulphan, Aspartam, NatriumCyclamat, Stevioside, Thaumatin, Sucrose, Lactose, Maltose oder Fructose, Glycyrrhizin u.a..

Als Antizahnstein-Wirkstoffe können z.B. organische Phosphonate wie Azacycloheptan-2,2-diphosphonsäure-Dinatriumsalz oder 1-Hydroxyethan,1,1-diphosphonsäure-Tetranatriumsalz enthalten sein. Als Fluorverbindungen eignen sich z.B. Natriumfluorid oder Natriummonofluorophosphat (Na₂PO₃F).

Als Vitamine können z.B. Retinol, Panthenol oder ein Salz der Pantothensäure, Ascorbinsäure oder Gemische davon enthalten sein. In einer bevorzugten Ausführung ist zur Verbesserung der entzündungshemmenden Eigenschaften eine Kombination von Panthenol und Retinol enthalten.

Weiterhin hat sich ein Gehalt an bestimmten gelösten zweiwertigen Metallsalzen, insbesondere an Salzen des Magnesiums und des Zinks, als wertvoll für die remineralisierenden Eigenschaften der erfindungsgemäßen Zahnreinigungsgele erwiesen. Geeignete Metallsalze sind z.B. die Sulfate, Fluoride, Citrate oder Acetate dieser Metalle. Bevorzugt sind gelöste Salze des Magnesiums oder Zinks oder einer Mischung beider Salze in einer Menge von 0,02, bis 0,2 Gew.-% Mg⁺⁺ und/oder Zn⁺⁺ enthalten.

Darüberhinaus können als weitere übliche Zahnpasteninhaltsstoffe z.B.
- Puffersubstanzen, z.B. primäre, sekundäre oder tertiäre Alkaliphosphate, Citronensäure/Na-Citrat
- wundheilende und entzündungshemmende Substanzen wie z.B. Harnstoff, Allantoin, Kamillenwirkstoffe wie Azulen, Alkali-Rhodamid, Acetylsalicylsäure-Derivate sowie
- niedere Alkohole wie z.B. Ethanol, Isopropanol
- Farbstofe und Pigmente
enthalten sein.

Die folgenden Beispiele sollen den Erfindungsgegenstand näher erläutern:

### Beispiele

Es wurden flüssige Zahnreinigungsgele der folgenden Zusammensetzung hergestellt:

| | 1 | 2 | 3 | 4 | 5 | V1 | V2 |
|---|---|---|---|---|---|---|---|
| Sident 8 | 12 | 12 | 12 | --- | --- | 12 | 12 |
| Zeodent 113 | --- | --- | --- | --- | 12 | --- | --- |
| RP-LA-2981 | --- | --- | --- | 12 | --- | --- | --- |
| Sorbit | 49 | 49 | 49 | 49 | 49 | 49 | 49 |
| Lipoxol 1550 | 1,5 | 1,5 | 1,5 | 1,5 | 1,5 | --- | --- |
| Lipoxol 400 | --- | --- | --- | --- | --- | 2,0 | 2,0 |
| Cekol 200 | 0,5 | 0,5 | 0,5 | 0,5 | 0,5 | 0,5 | 0,5 |
| Tagat S | 0,5 | 0,5 | 0,5 | 0,5 | 0,5 | 0,5 | 0,5 |
| Tego Betain BL 215 | 0,6 | 0,6 | 0,6 | 0,6 | 0,6 | 0,6 | 0,6 |
| Texapon K1296 | 1,5 | 1,5 | 1,5 | 1,5 | 1,5 | 1,5 | 1,5 |
| Mg SO₄·7H₂O | --- | 0,5 | 0,5 | --- | --- | --- | 0,5 |
| ZnSO₄·7H₂O | --- | 0,16 | --- | 0,16 | 0,16 | --- | 0,16 |
| Na₂PO₃F | 1,1 | 1,1 | 1,1 | 1,1 | 1,1 | 1,1 | 1,1 |
| Na₂HPO4 | 0,2 | 0,2 | 0,2 | 0,2 | 0,2 | 0,2 | 0,2 |
| Na-Saccharinat | 0,2 | 0,2 | 0,2 | 0,2 | 0,2 | 0,2 | 0,2 |
| Aromaöl | 1,0 | 1,0 | 1,0 | 1,0 | 1,0 | 1,0 | 1,0 |
| Triclosan | 0,1 | 0,1 | 0,1 | 0,1 | 0,1 | 0,1 | 0,1 |
| Ethanol | 2,0 | 2,0 | 2,0 | 2,0 | 2,0 | 2,0 | 2,0 |
| Wasser | ad 100 | ad 100 | ad 100 | ad 100 | ad 100 | ad 100 | ad 100 |
| Transparenz | 1 | 1 | 2 | 2 | 2 | 3 | 4 |

Die Transparenz wurde visuell wie folgt beurteilt:
1: klar bis hochtransparent
2: transparent
3: wenig transparent bis leicht trüb
4: trüb

Es wurden die folgenden Handelsprodukte verwendet:

| | |
|---|---|
| Sident 8: | Hydrogel-Kieselsäure |
| (Degussa-Hüls) | BET-Oberfläche: 60 m²/g |
| Zeodent 113: | Hydrogel-Kieselsäure |
| (Huber Corp.) | BET-Oberfläche: 150-250 m²/g |
| Kieselsäure RP-LA-2981: | Fällungs-Kieselsäure der Fa. Rhone Poulenc |
| (Rhone Poulenc) | |
| Cekol: 2000: | Natrium-Carboxy-methylcellulose |
| (Metsa) | Viskosität (2%-ig, 25°C): 1,3-2,3 Pa·s |
| Tagat S: | PEG30 Glyceryl Setearat |
| (Goldschmidt) | |
| Tego Betain BL 215: | Fettsäureamidoalkylbetain (30%-ig) |
| (Goldschmidt) | (Cocoamidopropyl Betain) |
| Texapon K 1296: | Natrium-Laurylsulfat (Pulver) |
| (Cognis Deutschland) | Aniontensidgehalt min. 94 Gew.-% |
| Lipoxol 1550: | Polyethylenglycol; MG=1550 |
| (RWE / DEA) | |
| Lipoxol 400: | Polyethylenglycol. MG=400 |
| RWE / DEA | |

## Patentansprüche

1. Wäßriges, flüssiges Zahnreinigungsgel mit einer Viskosität unterhalb 50 Pa·s (20°C) **gekennzeichnet durch** einen Gehalt von
| | |
|---|---|
| 10-20 Gew.-% | Kieselsäure-Poliermitteln |
| 40-60 Gew.-% | einer Feuchthaltemittel-Kombination, bestehend aus (jeweils bezogen auf das gesamte Mittel) weniger als 50 Gew.-% Sorbit weniger als 10 Gew.-% Glycerin und 0,1-5 Gew.-% Polyethylenglycol mit einem mittleren Molekulargewicht von 800-10 000 |
| 0,1-1 Gew.-% | eines antimikrobiellen Wirkstoffs, ausgewählt aus Triclocsan, Hexetidin und Gemischen davon |
| 1-10 Gew.-% | weiterer Zahnpasteninhaltsstoffe und |
| 25-35 Gew.-% | Wasser. |

2. Flüssiges Zahnreinigungsgel gemäß Anspruch 1, **dadurch gekennzeichnet, daß** als weitere Inhaltsstoffe ein wasserlösliches Bindemittel und eine Tensidkombination aus anionischen, zwitterionischen und nichtionischen Tensiden enthalten sind.

3. Flüssiges Zahnreinigungsgel gemäß einem der Ansprüche 1 oder 2, **dadurch gekennzeichnet, daß** als weiterer Inhaltsstoff ein gelöstes Salz des Magnesiums oder Zinks oder einer Mischung beider Salze in einer Menge von 0,02 bis 0,2 Gew.-% Mg⁺⁺ und/oder Zn⁺⁺ enthalten ist.

## Claims

1. Aqueous, liquid tooth cleaning gel with a viscosity below 50 Pa·s (20°C) **characterized by** a content of
| | |
|---|---|
| 10-20% by weight | of silica polishing agents |
| 40-60% by weight | of a humectant combination consisting of (in each case based on the total agent) less than 50% by weight of sorbitol less than 10% by of weight of glycerol and 0.1-5% by weight of polyethylene glycol with an average molecular weight of 800-10 000 |
| 0.1-1% by weight | of an antimicrobial active ingredient selected from triclosan, hexetidine and mixtures thereof |
| 1-10% by weight | of further toothpaste ingredients and |
| 25-35% by weight | of water. |

2. Liquid tooth cleaning gel according to Claim 1, **characterized in that** a water-soluble binder and a surfactant combination of anionic, zwitterionic and nonionic surfactants are present as further ingredients.

3. Liquid tooth cleaning gel according to one of Claims 1 or 2, **characterized in that** a dissolved salt of magnesium or of zinc, or a mixture of both salts in an amount of from 0.02 to 0.2% by weight Mg⁺⁺ and/or Zn⁺⁺ is present as further ingredient.

## Revendications

1. Gel liquide aqueux de nettoyage des dents ayant une viscosité inférieure à 50 Pa.s (20 °C), **caractérisé par** une teneur de
10 à 20 % en poids d'agents de polissage à base d'acide silicique
40 à 60 % en poids d'une combinaison d'agents humidifiants, constitués de (toujours par rapport à l'ensemble du produit)
moins de 50 % en poids de sorbitol
moins de 10 % en poids de glycérine et
0,1 à 5 % en poids de polyéthylèneglycol ayant un poids moléculaire moyen de 800 à 10 000
0,1 à 1 % en poids d'une substance active antimicrobienne choisie parmi le Triclosan, l'Hexétidine et leurs mélanges,
1 à 10 % en poids d'autres constituants de pâtes dentifrices et
25 à 35 % en poids d'eau.

2. Gel liquide de nettoyage des dents selon la revendication 1, **caractérisé en ce qu'**il contient comme autres constituants un liant soluble dans l'eau et une combinaison tensioactive faite d'agents tensioactifs anioniques, zwitterioniques et non ioniques.

3. Gel liquide de nettoyage des dents selon l'une des revendications 1 ou 2, **caractérisé en ce qu'**il contient comme autre constituant un sel dissous de magnésium ou de zinc, ou un mélange des deux sels en une quantité de 0,02 à 0,2 % en poids de Mg (++) et/ou Zn (++).
